(19) Europäisches Patentamt
European Patent Office
Office européen des brevets

(11) EP 2 594 247 A2

(12) # EUROPEAN PATENT APPLICATION

(43) Date of publication:
**22.05.2013 Bulletin 2013/21**

(51) Int Cl.:
***A61K 8/34*** *(2006.01)* ***A61Q 5/00*** *(2006.01)*
***A61K 8/81*** *(2006.01)*

(21) Application number: **12177613.2**

(22) Date of filing: **24.07.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.09.2011 US 201161530589 P**

(71) Applicant: **The Procter & Gamble Company**
**Cincinnati, OH 45202 (US)**

(72) Inventors:
- **Flohr, Andreas**
  **61476 Kronberg im Taunus (DE)**
- **Kripp, Thomas**
  **64407 Fränkisch-Crumbach (DE)**
- **Motley, Curtis**
  **West Chester, OH Ohio 45069 (US)**

(74) Representative: **Marollé, Patrick Pierre Pascal**
**Procter & Gamble Service GmbH**
**Patent Department**
**Berliner Allee 65**
**64295 Darmstadt (DE)**

(54) **Personal care composition comprising butylated hydroxyanisole compound**

(57) A personal care composition which has an ethylenic monomer having a molecular weight of 500 g/mole or less and a compound selected from the group consisting of 2-*tert*-butyl-4-hydroxy-anisole, 3-*tert*-butyl-4-hydroxy-anisole, and mixtures thereof. The compound is present in the personal care composition in an amount of from about 1 milligram to about 1000 milligram per kilogram of the ethylenic monomer.

EP 2 594 247 A2

Printed by Jouve, 75001 PARIS (FR)

**Description**

FIELD OF THE INVENTION

The present invention relates to a personal care composition comprising: (a) an ethylenic monomer having a molecular weight of 500 g/mole or less; (b) a compound selected from the group consisting of: 2-*tert*-butyl-4-hydroxy-anisole, 3-*tert*-butyl-4-hydroxy-anisole, and mixtures thereof; wherein (b) is present in the composition in an amount of from about 1 milligram to about 1000 milligram per kilogram of (a).

BACKGROUND OF THE INVENTION

Ethylenic compounds are commonly used in personal care products. For example, ethylenic monomers are known for their use in hair styling products. WO2009/088520A1 describes the use of ethylenic monomers to chemically modify the internal region of the hair shaft - in particular the ethylenic molecules may bond to the hair and/or to each other to form larger molecules. This, in turn, internally increases the rigidity of the hair, which allows style formation or increased volume and style retention longer periods of time.

Ethylenic compounds often need to be stabilised in order to inhibit reactions e.g. polymerisation, prior to use. 4-methoxy phenol, for example, is a known inhibitor of vinyl and acrylic monomers. In modem times, consumers of personal care products show greater and greater interest in the origin of the product's contents and any potential effect of such contents on human wellbeing.

Hence, there is a constant need for inhibitor compounds that are more consumer acceptable and with at least parity performance compared to known inhibitor compounds. Furthermore, there is a need for inhibitor compounds with greater inhibition efficacy. Moreover, there is a desire for improved inhibitors, which when inhibition is no longer desired e.g. when a reaction initiator is added, such that the ethylenic monomer has improved reactivity.

SUMMARY OF THE INVENTION

According to a first aspect, the present invention relates to a personal care composition comprising: (a) an ethylenic monomer having a molecular weight of 500 g/mole or less; (b) a compound selected from the group consisting of: 2-*tert*-butyl-4-hydroxy-anisole, 3-*tert*-butyl-4-hydroxy-anisole, and mixtures thereof; wherein (b) is present in the composition in an amount of from about 1 milligram to about 1000 milligram per kilogram of (a).

According to a second aspect, the present invention relates to a method of treating hair and/or skin comprising applying the composition, according to the first aspect, onto hair and/or skin.

According to a third aspect, the present invention relates to a kit comprising:

(i) a composition, according to the first aspect;
(ii) an oxidising formulation;

According to a fourth aspect, the present invention relates to the use of the composition, according to the first aspect, for treating hair and/or skin.

BRIEF DESCRIPTION OF THE DRAWINGS

Figure 1: Shows that an increase in molecular weight polymer occurs when the composition of the present invention is utilised compared to a comparative composition, as measured by gel permeation chromatography (GPC).
Figure 2: Shows the disappearance of ethylenic monomer over time as measured by proton nuclear magnetic resonance spectroscopy ($^1$H-NMR).

DETAILED DESCRIPTION OF THE INVENTION

In all embodiments of the present invention, all percentages are by weight of the total composition, unless specifically stated otherwise. All ratios are weight ratios, unless specifically stated otherwise. All ranges are inclusive and combinable. The number of significant digits conveys neither a limitation on the indicated amounts nor on the accuracy of the measurements.

All numerical amounts are understood to be modified by the word "about" unless otherwise specifically indicated. Unless otherwise indicated, all measurements are understood to be made at 25°C and at ambient conditions, where

"ambient conditions" means conditions under about one atmosphere of pressure and at about 50% relative humidity. All such weights as they pertain to listed ingredients are based on the active level and do not include carriers or byproducts that may be included in commercially available materials, unless otherwise specified.

Herein, "comprising" means that other steps and other ingredients which do not affect the end result can be added. This term encompasses the terms "consisting of" and "consisting essentially of". The compositions, methods, uses, kits, and processes of the present invention can comprise, consist of, and consist essentially of the elements and limitations of the invention described herein, as well as any of the additional or optional ingredients, components, steps, or limitations described herein.

The term "substantially free from" or "substantially free of" as used herein means less than about 1%, preferably less than about 0.8%, more preferably less than about 0.5%, still more preferably less than about 0.3%, most preferably about 0%, by total weight of the composition or formulation.

"Hair," as used herein, means mammalian hair including scalp hair, facial hair and body hair, more preferably hair on the human head and scalp. "Hair shaft" means an individual hair strand and may be used interchangeably with the term "hair."

"Internal region of the hair shaft," as used herein, means any non-surface portion of the hair shaft, including the inner portion of the cuticle and underneath the cuticle. "Non-surface portion" may be understood to mean that portion of the hair that is not in direct contact with the outside environment.

"Proximal to the scalp," as used herein, means that portion of an extended, or substantially straightened, hair shaft that is closer in distance to the scalp than to the end of the hair. Thus, about 50% of the hair would be considered proximal to the scalp, and about 50% of the hair would be distal to the scalp. "z cm proximal to the scalp" means a distance "z" along the hair, with one endpoint being on or directly adjacent to the scalp, and the second endpoint being measured "z" centimetres along the length of the extended or substantially straightened hair.

"Cosmetically acceptable," as used herein, means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives," as used herein, includes but is not limited to, amide, ether, ester, amino, carboxyl, acetyl, acid, salt and/or alcohol derivatives of a given compound.

"Monomer," as used herein, means a discrete, non-polymerised chemical moiety capable of undergoing polymerisation in the presence of an initiator.

"Ethylenic monomer," as used herein, means a chemical species that contains an olefinic carbon-carbon double bond (C=C) and is capable of undergoing polymerization in the presence of an initiator.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

The term "hairstyling polymer" as used herein means hair-fixing polymers which form films on a surface. In the context of hair, this surface is the surface of individual hair fibres or a plurality thereof. The polymer causes them to be glued together to build welds, which are crosslinks that provide the hold benefit. In concert, these welds form a 'hairnet' to provide hair hold and volume benefits to the user. When the net of welds is effectively formed, the hold and volume benefits can last all day and offer good resistance to environmental humidity.

The term "molecular weight" or "M.Wt." as used herein refers to the number average molecular weight unless otherwise stated.

"Chemically modify," or grammatical equivalents thereof, as used herein, means that a chemical moiety such as monomer and/or crosslinker and/or polymer, stably affixes to a second chemical moiety, for example, a keratin protein, another component of hair, and/or another monomer or crosslinker or polymer.

"Separately packaged," as used herein, means any form of packaging that prevents one composition or formulation from coming into physical contact, or admixing, with a second composition or formulation. "Separately packaged" may mean that the individual compositions/formulations are packaged in separate containers, or alternatively in a single container partitioned such that the compositions/formulations are not in physical contact.

"Relative humidity", as used herein, means the amount of water vapour carried in the air. "High relative humidity" herein refers to a relative humidity higher than that at ambient conditions i.e. at least about 70%. "Environmental humidity" as used herein relates to relative humidity that the consumer may be exposed to once the method of the present invention has been completed. An example of environmental humidity is that due to weather conditions. "Environmental humidity resistance", as used herein relates to the ability of the hair to better resist negative consequences of environmental

humidity.

"Increased style retention" and/or "hairstyle durability," as used herein, means that the hair style formed, shaped, and/or obtained after application of the composition of the present invention to the hair is maintained for a longer period of time relative to hair of the same being to which no composition has been applied.

"Increased appearance of volume," as used herein, means that the hair exhibits a visually noticeable greater volume, i.e., distance between the scalp and the outermost layer of hair style and/or distance between individual hairs, after application of a composition of the present invention relative to before a composition was applied.

"Increased resistance to moisture," as used herein, means that after application of a composition of the present invention, the hair fails to exhibit visually noticeable effects, such as loss of volume, loss of style, increase in frizz, etc. upon exposure to water vapour (i.e., relative humidity greater than about 50%) relative to before a composition has been applied.

"Kit," as used herein, means a packaging unit comprising a plurality of components. An example of a kit is, for example, a first composition and a separately packaged second composition. Another kit may comprise a first composition and an energy delivery device. A different kit may comprise three different types of separately packaged composition and a hair styling implement. A further kit may comprise application instructions comprising a method and a composition/formulation.

"Separately packaged," as used herein, means any form of packaging that prevents a first composition from coming into physical contact, or admixing, with a second composition. "Separately packaged" may mean that the individual compositions are packaged in separate containers, or alternatively in a single container partitioned such that the compositions are not in physical contact.

"Implement," as used herein, means a device used to facilitate application of a composition to the hair and/or manipulation of the hair. Examples of implements include, but are not limited to, a comb, a means for directed delivery (e.g., an applicator or tube), a covering for the hair (e.g., plastic bag, shower cap, etc.), and combinations thereof.

"Energy delivery device," as used herein, means any device used to deliver energy to keratinous tissue, including the hair and scalp. "Delivery of energy," means that the surface of the keratinous tissue is exposed to the energy emanating from the energy delivery device, where it may penetrate to the desired layers of the tissue, including the hair shaft and/or hair follicle. Energy includes but is not limited to energy in the form of light, heat, sound (including ultrasonic waves), electrical energy, magnetic energy, electromagnetic energy (including radiofrequency waves and microwaves), and combinations thereof.

The present invention provides a composition comprising an ethylenic monomer that is more effectively stabilised by use of a polymerisation inhibitor. By choosing a compound selected from the group consisting of: 2-*tert*-butyl-4-hydroxy-anisole, 3-*tert*-butyl-4-hydroxyanisole, and mixtures thereof (also known as compound [b] herein), excellent stability for the ethylenic monomer is provided versus other inhibitors. Despite the fact that the compound (b) has better stabilisation efficacy, reactivity of the ethylenic monomer is not compromised (see Figures 1 and 2). It is known in the art that resulting polymer molecular weight is inversely proportional to the square root of the initial radical concentration - a radical in this sense being an ethylenic monomer having been activated by an initiator. In correlation with the improved inhibition effect, fewer radicals are generated upon initiation of polymerisation, thus leading to a tendency for higher molecular weights of polymer (see Figure 1). Furthermore, upon exposure to elevated temperatures, compound (b) more is more stable than all other inhibitors tested and for a longer time.

Without being bound by theory, it is believed that upon the formation of a carbon radical in place of the $-CH=CH_2$ group on the ethylenic monomer, the compound (b) is able to donate a proton from its hydroxyl group to the radical on the ethylenic monomer. The resulting monomer is unable to polymerise since a radical is no longer present. It is also believed that the butyl group on the inhibitor compound acts as an electron-donating (+I) group able to stabilise the resulting radical in the phenyl ring. This is explained more precisely hereinafter. Formula I shows 3-*tert*-butyl-4-hydroxyanisole (CAS No.: 121-00-6), wherein the methoxy group is represented by "OMe" and the tert-butyl group by "Bu-t". As used herein, the carbon-1 position for the herein mentioned anisole-based compounds is the carbon substituted with the methoxy group.

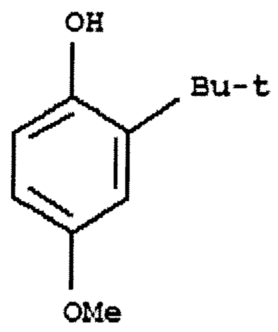

Formula I

An example of an ethylenic monomer is 3-sulfopropacrylate (3-SPA). It will, over time, generate radicals at the vinyl position that can react with another monomer and prematurely polymerise and thus needs to be stabilized to avoid premature polymerisation. Ethylenically unsaturated monomers such as 3-SPA can be abbreviated "R" (see Formula II).

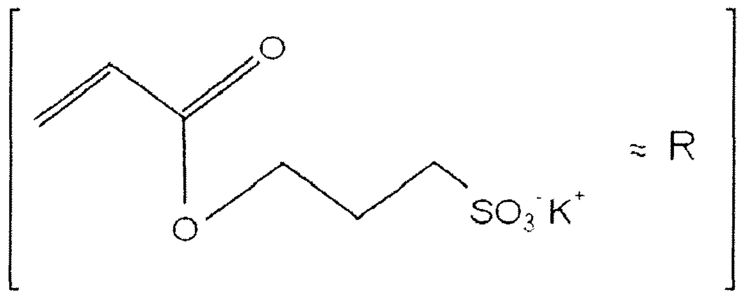

Formula II

Once an ethylenic monomer radical (R·) is created, it reacts with inhibitor compound (b). Inhibitors generally form stabile, thus unreactive radicals, and hence prevent the monomer radical from premature auto-polymerisation. When reacting with compound (b), the monomer radical abstracts hydrogen from the hydroxyl group, and thus gets regenerated (see Scheme I).

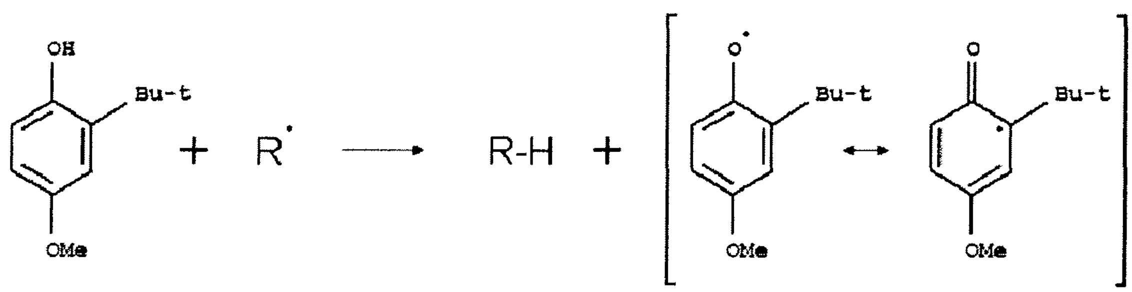

Scheme I

Without wanting to be bound by theory it is believed that compound (b) is a more effective inhibitor for the polymerisation of ethylenically unsaturated monomers compared to 4-methoxy phenol, for example, because it forms more stable intermediates. The tert-butyl group of compound (b) has a positive electron inducing effect for the ring radical formed upon hydrogen abstraction, thus is more likely to form for compound (b) than for 4-methoxy phenol (see Scheme II).

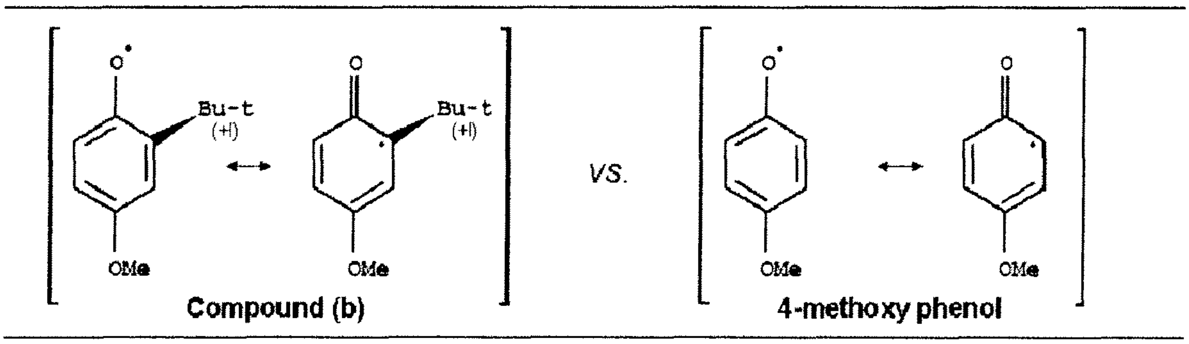

Scheme II

According to the first aspect, the composition comprises an ethylenic monomer having a molecular weight of about 500 g/mole or less. In an embodiment, the ethylenic monomer comprises a vinyl group. "Vinyl group" as used herein, means $H_2C=CH-R$. In an embodiment, the ethylenic monomer comprises an acrylate group or a methacrylate group. "Acrylate group" as used herein, means $H_2C=CH-C(O)O-R$. Acrylic acid, for example, comprises an acrylate group since R is hydrogen. "Methacrylate group" as used herein means $H_2C=C(CH_3)-C(O)O-R$. In an embodiment, the ethylenic monomer is selected from the group consisting of: mesaconic acid, 2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2-methylenebutyrate, methacryloyl-L-lysine, N-(2-hydroxypropyl)methacrylamide, 2-acrylamidodiglycolic acid, 2-ethoxyethyl acrylate, 2-butoxyethyl acrylate, N-isopropylmethacryalmide, 2-aminoethyl methacrylate, 2-bromoethyl acrylate, 3-(dimethylamino)propyl acrylate, (3-acrylamidopropyl)trimethyl ammonium salt, [2-(acryloyloxy)ethyl]-trimethylammonium salt, alkylacetamidoacrylate, sulfoalkyl (meth)acrylate, 3-sulfopropyl acrylate, 3-sulfopropyl methacrylate, derivatives thereof, and mixtures thereof. In an embodiment, the composition comprises at least one ethylenic monomer, selected from the group cited above, as the sole ethylenic monomer(s). In an embodiment, the ethylenic monomer is selected from the group consisting of 3-sulfopropyl acrylate, 3-sulfopropyl methacrylate, and salts, derivatives and mixtures thereof. In an embodiment, the only ethylenic monomer(s) present are selected from the group consisting of 3-sulfopropyl acrylate, 3-sulfopropyl methacrylate, and salts, derivatives thereof, and mixtures thereof. In an embodiment, the sole ethylenic monomer is 3-sulfopropyl acrylate. In an embodiment, the ethylenic monomer is 3-sulfopropyl acrylate, which is added to the composition as 3-sulfopropyl acrylate potassium salt.

In another embodiment, the ethylenic monomer is selected from the group consisting of: acrylic acid, sodium acrylate, potassium acrylate, calcium acrylate, monoethanolamine acrylate, 3-hydroxypropyl acrylate, 2,5-butylaminoethyl acrylate, methacrylic acid, sodium methacrylate, potassium methacrylate, calcium methacrylate, monoethanolamine methacrylate, 2-N,N-dimethylaminoethyl acrylate, glycidyl methacrylate, 2-dimethylamino ethyl methacrylate, 2-hydroxypropyl methacrylate, 3-hydroxypropyl methacrylate, 2,4-dihydroxybutyl methacrylate, 2,3-epoxybutyl methacrylate, 2-t-butylaminoethyl methacrylate, 2-(2-diethylamino)ethyl methacrylate, ethylene glycol mono methacrylate, itaconic acid (and salts thereof), vinyl pyridine, resorcinol, and mixtures thereof.

In an embodiment, the molecular weight of the ethylenic monomer is important because of the need for the monomer to penetrate into the hair shaft prior to polymerisation. Large and/or bulky monomers would penetrate less easily into the hair shaft. In an embodiment, the ethylenic monomer has a molecular weight of from about 50 g/mole to about 500 g/mole, or from about 75 g/mole to about 400 g/mole, or from about 100 g/mole to about 400 g/mole, or from about 150 g/mole to about 300 g/mole. In an embodiment, the ethylenic monomer does not have a molecular weight of below about 50 g/mole, or below about 75 g/mole, or below about 100 g/mole, or below about 150 g/mole, or above about 500 g/mole, or not above about 400 g/mole, or not above about 300 g/mole.

The ethylenic monomer may be present in the composition in an amount of from about 0.1% to about 20%, or from about 1% to about 15%, or from about 5% to about 14%, or from about 7% to about 13%, or about 11% to about 12.5%, by total weight of the composition.

In an embodiment, two or more different ethylenic monomers are present in the composition pursuant to the first aspect. The resultant polymers may be copolymers.

The composition comprises a compound selected from the group consisting of: 2-*tert*-butyl-4-hydroxy-anisole, 3-*tert*-butyl-4-hydroxy-anisole, and mixtures thereof. Furthermore, (b) is present in the composition in an amount of from about 1 milligram to about 1000 milligram per kilogram of (a), wherein (b) means the total amount of compound selected from the group consisting of: 2-*tert*-butyl-4-hydroxy-anisole, 3-*tert*-butyl-4-hydroxy-anisole, and mixtures thereof, and wherein (a) means the total amount of ethylenic monomer having a molecular weight of about 500 g/mole or less. This

'total amount' also applies where the composition comprises a specific ethylenic monomer. In an embodiment, (b) is present in the composition in an amount of from about 50 milligram to about 800 milligram, or from about 100 milligram to about 500 milligram, or from about 100 milligram to about 300 milligram, or from about 100 milligram to about 200 milligram, per kilogram of (a). In an embodiment, (b) is the mixture of 2-*tert*-butyl-4-hydroxy-anisole and 3-*tert*-butyl-4-hydroxy-anisole. In an embodiment, the mixture of 2-*tert*-butyl-4-hydroxy-anisole and 3-*tert*-butyl-4-hydroxy-anisole comprises greater than 50 wt%, or greater than 60 wt%, or greater than 80 wt% 3-*tert*-butyl-4-hydroxy-anisole.

The compound (b) functions as a polymerisation inhibitor i.e. it stabilises the ethylenic monomer so that it does not polymerise. Compound (b) provides excellent performance in that the ethylenic monomer is effectively stabilised and also when the ethylenic monomer exposed to an initiator, the ethylenic monomer polymerises efficiently and quickly. In an embodiment, the composition is substantially free of a polymer derived from the polymerisation of ethylenic monomers.

Other polymerisation inhibitors used for stabilising ethylenic monomers are known in the art e.g. 4-methoxy phenol. In an embodiment, the composition comprises less than about 500 ppm, or less than about 400 ppm, or less than about 300 ppm, or less than about 200 ppm, or less than about 100 ppm, or less than about 50 ppm, or less than about 10 ppm, of 4-methoxy phenol. In an embodiment, the composition is substantially free of a polymerisation inhibitor, with the exception of compound (b).

The composition may comprise a crosslinker having a molecular weight suitable to penetrate the hair shaft. The purpose of the crosslinker is to covalently bond the monomer to the hair, and/or monomer to other monomer, and/or and polymer to other polymer. The molecular weight of the crosslinker may be about 500 g/mole or less, or from about 100 g/mole to about 500 g/mole, or from about 100 g/mole to about 400 g/mole, or from about 200 g/mol to about 400 g/mole.

The crosslinkers may be selected from the group consisting of: 1,4-bisacryloylpiperazine, methylenebisacrylamide, ethylenebisacrylamide, divinylbenzene, poly-ethyleneglycol di(meth)acrylate, ethylene glycol di(meth)acrylate, 1,3-butanediol di(meth)acrylate, 1,4-butanediol di(meth)acrylate, neopentyl glycol di(meth)acrylate, bis[2-(methacryloyloxy)ethyl] phosphate, N,N'-bis(acryloyl)cystamine, N,N-diallylacryalmide, triallyl cyanurate, 3-(acryloyloxy)-2-hydroxypropyl methacrylate, and mixtures thereof.

In an embodiment, the ratio of the weight percentage of the ethylenic monomer to the weight percentage of the crosslinker (i.e. ethylenic monomer:crosslinker) is from about 50:1 to about 10:1, or from about 40:1 1 to about 10: 1, or from about 20:1 1 to about 10:1.

A composition or formulation as described herein, or a plurality thereof, may further comprise a viscosity-increasing agent. Viscosity is important when the composition is in the form of gel, cream, lotion, emulsion etc because it prevents the composition from sliding off the skin and/or hair. However, lower viscosities allow actives to penetrate/ diffuse more easily e.g. into the internal region of the hair shaft. The viscosity of the composition when it is in the form of a gel is: from about 500 mPa·s to about 15000 mPa·s, or from about 1000 mPa·s to about 10000 mPa·s, or from about 1500 mPa·s to about 7500 mPa·s, or from about 2000 mPa·s to about 5000 mPa·s, measured with a Brookfield Viscosimeter RVDV III Ultra CP 52 at 25 °C and 1 rpm.

The viscosity-increasing agent may be selected from the group consisting of non-ionic thickeners, cationic thickeners, anionic thickeners, amphoteric thickeners, and mixtures thereof; preferably non-ionic thickeners, anionic thickeners, and mixtures thereof. The viscosity-increasing agent may be present in the composition in an amount of from about 0.1 % to about 10%, or from about 0.2% to about 5.0%, by total weight of the composition.

Non-ionic or anionic thickeners (or mixtures thereof) are preferred for the composition due to the typically anionic chemistry of any polymerised ethylenic monomer. Non-ionic or anionic thickeners are less likely to interact directly with any formed polymer and hence the formation of insoluble complexes or precipitates is also less likely. The viscosity-increasing agent is also preferably stable at the required pH and does not substantially affect the active levels of ethylenic monomer. The viscosity-increasing agent may be a cross-linked or a non-crosslinked polymer.

In an embodiment, the viscosity-increasing agent is a hydrophobically-modified polyacrylate polymer. Such hydrophobically-modified polyacrylate polymers are particularly suitable when the composition/formulation is created by the addition of at least one salt. The composition may comprise from about 0.5% to about 1.5% of the hydrophobically-modified polyacrylate polymer, by total weight of the composition. Suitable hydrophobically-modified polyacrylate polymers include: acrylates/C10-C30 alkylacrylates copolymers such as Ultrez® 20/21 from Lubrizol, and Permulen® TR1 from Lubrizol; acrylates/beheneth-25 methacrylate copolymers such as Aculyn® 28 from Rohm & Haas; acrylates/ceteth-20 itaconate copolymers such as Structure® 3001 or 2001 from Akzo Nobel.

In an embodiment, the viscosity-increasing agent is a non-crosslinked associative thickening polymer. The composition may comprise from about 0.5% to about 3% of the non-crosslinked associative thickening polymer, by total weight of the composition. Suitable associative thickeners include polyurethane-based polymers such as polyurethane-30 e.g. LuvigelSTAR® from BASF. Also EO-PO-block copolymers may be useful, for example Pluronics® from BASF.

In an embodiment, the viscosity-increasing agent comprises at least one polysaccharide, preferably at least one heteropolysaccharide. The total polysaccharide present in the composition may be from about 0.2% to about 5%,

or from about 0.5% to about 4%, by total weight of the composition. Suitable polysaccharides and heterosaccharides include starches and derivatives thereof, e.g. mono- or di-esters with phosphoric acid, cellulose types and their derivatives, xanthan gums, carrageenans. Preferred heteropolysaccharides include xanthan gum such as Keltrol® T from Kelco, and Natrosol® 250 HHR from Herkules. In an embodiment, the polysaccharide is selected from the group consisting of hydroxyethylcellulose, hydroxypropylcellulose, xanthan gum, carrageenans, and mixtures thereof. Xanthan gums and derivatives thereof are present in an amount of from about 0.2% to about 1.5%, or from about 0.5% to about 0.9%, by total weight of the composition. Starches and derivatives thereof are present in an amount of from about 3% to about 4% by total weight of the composition. A preferred starch is hydroxypropyl starch phosphate such as Structure® XL from National Starch. In an embodiment, the composition comprises two different polysaccharide viscosity-increasing agents.

The composition may further comprise a cosmetically acceptable carrier. Another composition or formulation as described herein, or a plurality thereof, may also comprise a cosmetically acceptable carrier. The composition/formulation may comprise from about 60% to about 99.9%, or from about 70% to about 95%, or from about 80% to about 90%, of a cosmetically acceptable carrier, by total weight of the composition or formulation. The cosmetically acceptable carrier may comprise water; silicones such as volatile silicones, amino or non-amino silicone gums; organic compounds such as $C_2$-$C_{10}$ alkanes, acetone, methyl ethyl ketone, volatile organic $C_1$-$C_{12}$ alcohols, esters of $C_1$-$C_{20}$ acids and of $C_1$-$C_8$ alcohols such as methyl acetate, butyl acetate, ethyl acetate, and isopropyl myristate, dimethoxyethane, diethoxyethane, $C_{10}$-$C_{30}$ fatty alcohols such as lauryl alcohol, cetyl alcohol, stearyl alcohol, and behenyl alcohol; $C_{10}$-$C_{30}$ fatty acids such as lauric acid and stearic acid; $C_{10}$-$C_{30}$ fatty amides such as lauric diethanolamide; $C_{10}$-$C_{30}$ fatty alkyl esters such as $C_{10}$-$C_{30}$ fatty alkyl benzoates; hydroxypropylcellulose, and mixtures thereof. In an embodiment, the carrier comprises water, fatty alcohols, volatile organic alcohols, and mixtures thereof. In an embodiment the carrier is water.

In an embodiment, the composition further comprises a cation and an anion; wherein the cation is selected from the group consisting of inorganic cations having a charge density of 0.05 charge/picometre or more, or about 0.052 charge/picometre or more. In an embodiment the inorganic cation is a metal. In an embodiment, the cation may not be selected from inorganic cations having a charge density of about 0.04 charge/picometre or less, or less than about 0.05 charge/picometre. In an embodiment, the cation is selected from the group consisting of inorganic cations having a charge density of about 0.050 charge/picometre to about 0.090 charge/picometre, or from about 0.052 charge/picometre to about 0.080 charge/picometre, or to about 0.070 charge/picometre, or to about 0.060 charge/picometre, or to about 0.053 charge/picometre.

The charge density of an ion is calculated by dividing the charge by the ionic radius which results in charge per picometre, the charge density. For example, $Sr^{2+}$ has an ionic radius of 127 pm and a charge of 2. Consequently, the charge per picometre is 0.0157 charge/pm. Ion radii of common metals can be found in Table I of EP patent application 11151384.2 filed on 19th January 2011 in the name of The Procter & Gamble Company where the named inventors are A. Flohr, T. Krause, and M. Loifenfeld and the application is entitled COMPOSITION FOR CHEMICALLY MODIFYING THE INTERNAL REGION OF A HAIR SHAFT. Tables of ionic radii can also be found in common inorganic chemistry textbooks, for example Atkins P.W., Physical Chemistry, 6th Edition, 2001.

In an embodiment, the inorganic cation has a charge of at least 2+, or at least 3+. In an embodiment, the inorganic cation is not a transition metal. In an embodiment, the inorganic cation is not a metal capable of cleaving hydrogen peroxide. In a preferred embodiment, the inorganic cation is $Al^{3+}$.

The molar ratio of the cation to the monomer (i.e. cation:monomer) may be from about 1:10 to about 2:1, or from about 1:5 to about 3:2, or from about 1:2 to about 1:1, or from about 1:3 to about 1:1. The molar amount of cation is preferred to be in excess of the molar amount of counterion of the ethylenic monomer when the ethylenic monomer is added into the composition in the form of a salt.

The inorganic cation may also be present in an oxidising formulation, a finishing formulation, and/or a combination thereof. In an embodiment, the oxidising formulation comprises a cation, wherein the cation is not selected from inorganic cations having a charge density of about 0.04 charge/picometre or less, or less than about 0.05 charge/picometre. In an embodiment, the oxidising formulation comprises a cation, wherein the cation is selected from the group consisting of inorganic cations having a charge density of about 0.05 charge/picometre or more, and the cation is present in an amount of from about 0.001% to about 2%, or from about 0.01% to about 1%, or from about 0.04% to about 0.2%, by total weight of the oxidising formulation.

In an embodiment, the present invention comprises an anion. The anion may be selected from the group consisting of sulfate, sulfonate, phosphate, nitrate, chloride, citrate, lactate, formate, and mixtures thereof. Preferred anions are selected from the group consisting of sulfate, sulfonate, and mixtures thereof. In a most preferred embodiment, the anion is sulfate.

In an embodiment, the molar ratio of the cation to the anion (cation:anion) is from about 1:5 to about 5:1, or from about 1:4 to about 3:1, or from about 2:3 to about 3:2. Where the inorganic metal cation is $Al^{3+}$ and the anion is sulfate, the molar ratio of the cation to the anion is about 2:3. The anion may also be present in the oxidising formulation, a finishing formulation, and a combination thereof.

In an embodiment, the composition is for chemically modifying the internal region of the hair shaft. In an

embodiment, the chemically modifying is selected from the group consisting of: the formation of a polymer in the internal region of the hair shaft; the modification of the internal region of the hair shaft with a polymer; and combinations thereof. In an embodiment, the polymerisation that occurs is free radical polymerisation.

In an embodiment, the composition is substantially free of oxidising agents and/or initiators. In another embodiment, the composition is substantially free of oxidising agents selected from the group consisting of: peroxides, preferably hydrogen peroxide; persulfates, preferably potassium persulfate or sodium persulfate; and mixtures thereof. In another embodiment, the composition is substantially free of an alpha-methylene lactone compound. In another embodiment, the composition is in ready-to-use form, wherein ready-to-use form means that no pre-mixing is required prior to application onto hair. In another embodiment, the composition is substantially free of at least one of the following: a reducing agent, a transition metal, hair dye, or combinations thereof. In an embodiment, the composition is substantially free of a salt compound. In an embodiment, the composition is substantially free of a salt compound with the exception of the salt of the ethylenic monomer.

In an embodiment, the composition provides a performance benefit selected from the group consisting of: increased hair volume, increased hair fullness, increased hair bounciness, increased hair movement, improved hair feel, improved hair mobility, improved hair texture, improved hair appearance of health, increased control over hairstyle, improved desired hair shape retention, improved hairstyle durability, improved hair shine, more tamed hair, increased curl definition, more defined-looking hair, improved humidity resistance, and combinations thereof. In an embodiment, the performance benefit is still noticeable to at least about 40% of consumers after about 15 washes, or to at least about 45% of consumers, or to at least about 50% of consumers, or to at least about 55% of consumers, or to at least about 60% of consumers, or to at least about 65% of consumers, or to at least about 70% of consumers. In an embodiment, the consumers are selected from the group consisting of: consumers that regularly style their hair and have thin and/or limp hair, and consumers that regularly style their hair and have thick and/or unruly hair.

A composition or formulation as described herein, or a plurality thereof, may further comprise one or more optional components known or otherwise effective for use in hair care or personal care products, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004, both of which are incorporated by reference herein in their entirety. Some non-limiting examples of such optional components are disclosed below, and include plasticizers, surfactants (which may be anionic, cationic, amphoteric or nonionic), neutralizing agents, propellants, hair conditioning agents (e.g., silicone fluids, fatty esters, fatty alcohols, long chain hydrocarbons, cationic surfactants, etc.), emollients, lubricants and penetrants such as various lanolin compounds, vitamins, proteins, preservatives, dyes, tints, bleaches, reducing agents and other colorants, sunscreens, gelling agents, physiologically active compounds for treating the hair or skin (e.g., anti-dandruff actives, hair growth actives), non-polymeric thickeners including clays, and perfume.

Oily compounds may aid the penetration of the ethylenic monomer into the skin and/or scalp, which may not be preferred. In an embodiment, the composition is substantially free of oily compounds. In another embodiment, the composition and the oxidising formulation (described below) are substantially free of oily compounds.

The composition may be in different product forms. For example, it may be in a form selected from the group consisting of a gel, an emulsion, a cream, a spray, a lotion, or a mousse. The oxidising formulation may be in the form of gel, an emulsion, a cream, spray, lotion, mousse. The most preferred form for the oxidising formulation is a lotion.

The composition may have a pH of from about 2.0 to about 9.0. In an embodiment, the pH is from about 2.5 to about 7.5, from about 4.0 to about 7.0, or from about 4.0 to about 6.9. In an embodiment, the pH is from about 4.5 to about 6.7, from about 5.0 to about 6.6, from about 5.2 to about 6.5, from about 5.3 to about 6.5, from about 5.5 to about 6.5, or from about 5.5 to about 6.0. In an embodiment, the composition does not have a pH of about 7.0 or more.

According to the second aspect, the present invention relates to a method of treating hair and/or skin comprising applying the composition, according to the first aspect, onto hair and/or skin. In an embodiment, the hair and/or skin has been pre-treated with an oxidising formulation.

In an embodiment, the method comprises applying an oxidising formulation to the hair. The oxidising formulation comprises an oxidising agent. The oxidising agent may be selected from the group consisting of: peroxides, preferably hydrogen peroxide; persulfates, preferably potassium persulfate or sodium persulfate; and mixtures thereof. Another composition or formulation as mentioned herein, or a plurality thereof, may comprise at least one oxidising agent. In an embodiment, the composition according to the first aspect is substantially free of oxidising agent. In an embodiment, the oxidising formulation is substantially free of hair colouring agents, or substantially free of oxidative dyes and/or direct dyes.

The oxidising agent may be present in an amount of from about 0.01% to about 15%, by total weight of the oxidising formulation. When a persulfate oxidising agent is used, it may be in powder form and mixed as a liquid immediately prior to application onto hair. The final amount of persulfate in the composition/formulation may be from about 0.5% to about 2%, or 0.8% to about 1.2%, by total weight of the composition/formulation. When the oxidising agent is

a peroxide, the peroxide may be present in an amount of from about 0.5% to about 5%, or from about 1% to about 4%, or from about 1.3% to about 3%, or from about 1.5% to about 3%, by total weight of the composition or formulation.

In an embodiment, after applying the oxidising formulation to the hair but prior to de-wetting the hair, the oxidising formulation is allowed to remain on the hair for a period of time y, wherein time y is from about 1 min to about 120 min, or from about 2 min to about 45 min, or from about 3 min to about 20 min, or from about 4 min to about 10 min.

When the oxidising formulation comprises peroxide, the oxidising formulation may comprise a buffer system to stabilise the pH. Suitable buffers may also act as chelating agents. Chelation of transition metals, for example copper or iron from pipes which might be present in trace amounts in tap water, is important because peroxides are sensitive to cleavage by transition metals. In the absence of a buffer system the transition metal may cleave the peroxide, deactivating it. Typical buffer systems comprise a strong acid and its weak conjugate base or a weak base and its conjugate acid. An example of a suitable buffer system is phosphoric acid and disodium phosphate. Another example of a suitable buffer system is citric acid and sodium hydroxide. In an embodiment, the composition according to the first aspect comprises a buffer system.

The method may comprise de-wetting the hair, which occurs after application of the oxidising formulation and prior to the application of the composition according to the first aspect. In an embodiment the de-wetting the hair comprises the application of an absorbent material to the hair such that wetness is transferred from the hair to the absorbent material and wherein the wetness comprises the cosmetically acceptable carrier. The absorbent material may be selected from the group consisting of: towel, absorbent paper, and combinations thereof. In an embodiment, the de-wetting the hair comprises allowing moisture to evaporate from the hair wherein the moisture comprises the cosmetically acceptable carrier. In an embodiment, the de-wetting the hair comprises towel drying the hair such that the oxidising formulation no longer drips from the hair. In an embodiment, the de-wetting the hair comprises removing superficial oxidising formulation from the hair. In an embodiment, the de-wetting the hair does not comprise rinsing the oxidising formulation from the hair. The de-wetting the hair may last for time z, wherein time z is from about 1 min to about 120 min, or from about 2 min to about 45 min, or from about 3 min to about 20 min, or from about 4 min to about 10 min.

In an embodiment, the method comprises allowing the composition, according to the first aspect, to remain on the hair for a period of time x, wherein the time x is from about 1 min to about 120 min; rinsing the hair; washing the hair. The time x may be from about 5 mins to about 100 min, or from about 10 min to about 90 min, or from about 20 min to about 60 min. In an embodiment, the method comprises the application of an energy delivery device to the hair, which may be applied proximal to the scalp.

According to the third aspect, the present invention relates to a kit comprising:

(i) a composition, according to the first aspect;
(ii) an oxidising formulation.

In an embodiment, the kit comprises: (i) a composition, according to the first aspect; (ii) an oxidising formulation wherein the oxidising formulation is substantially free of a hair dye; (iii) optionally application instructions comprising the method according to the second aspect. In an embodiment, the kit further comprises the oxidising formulation according to the first aspect, which is packaged separately from the composition according to the first aspect. Another embodiment relates to a kit comprising:

(i) application instructions comprising the method according to the second aspect;
(ii) a product comprising the composition according to the first aspect;
(iii) a product comprising the oxidising formulation, wherein the oxidising formulation comprises from about 0.01% to about 15% oxidising agent, by total weight of the oxidising formulation;
(iv) optionally a product comprising a formulation being different from the composition according to the first aspect and different from the oxidising formulation, and wherein the formulation is a hair treatment agent selected from the group consisting of oxidising formulations, finishing formulations, reducing formulations, hairstyling formulations, finishing formulations, conditioning formulations, shampoo formulations, dyeing formulations, and combinations thereof.

In another embodiment of the kit, the kit further comprises one or more of the following:

(v) an implement;
(vi) a device.

According to the fourth aspect, the present invention relates to the use of the composition, according to the first aspect, for treating hair and/or skin. In an embodiment, the use is for modifying the internal region of a hair shaft.

EXAMPLES

Composition: Examples 1 - 7

| | Ex. 1 | Ex. 2 | Ex. 3 | Ex. 4 | Ex. 5 | Ex. 6 | Ex. 7 |
|---|---|---|---|---|---|---|---|
| Purified water | QSP | QSP | QSP | QSP | QSP | QSP | QSP |
| Hydroxyethylcellulose [1] | - | 0.20 | 0.20 | 0.20 | 0.20 | 0.20 | 0.20 |
| Hydroxyethylcellulose [6] | 0.70 | - | - | - | - | - | - |
| Disodium EDTA | 0.12 | 0.12 | 0.12 | 0.12 | 0.12 | 0.12 | 0.12 |
| Xanthan gum [2] | - | 0.50 | 0.50 | 0.50 | 0.50 | 0.50 | 0.50 |
| Aluminium sulfate octadecahydrate | - | 2.40 | - | - | - | - | - |
| Sodium hydroxide | - | 0.76 | 0.76 | 0.76 | 0.76 | 0.76 | 0.76 |
| Citric acid | 0.30 | 0.30 | 0.30 | 0.30 | 0.30 | 0.30 | 0.30 |
| 2-phenoxyethanol | 1.00 | 1.00 | 1.00 | 1.00 | 1.00 | 1.00 | 1.00 |
| p-hydroxybenzoic acid methylester | 0.20 | - | - | - | - | - | - |
| Methyl parabene | - | 0.20 | 0.20 | 0.20 | 0.20 | 0.20 | 0.20 |
| Coceth-10 [3] | 0.70 | 0.70 | 0.70 | 0.70 | 0.70 | 0.70 | 0.70 |
| PEG-35 castor oil [4] | 0.70 | 0.70 | 0.70 | 0.70 | 0.70 | 0.70 | 0.70 |
| Fragrance | 0.30 | 0.30 | 0.30 | 0.30 | 0.30 | 0.30 | 0.30 |
| 3-sulfopropyl acrylate potassium salt 5 | 8.00 | 10.00 | 12.00 | 12.00 | 12.00 | 12.00 | 15.00 |
| 2-*tert*-butyl-4-hydroxy-anisole | 750 ppm | 250 ppm | 180 ppm | - | 280 ppm | 10 ppm | 10 ppm |
| 3-*tert*-butyl-4-hydroxy-anisole | - | 670 ppm | 450 ppm | 950 ppm | 630 ppm | - | 270 ppm |
| Total | 100 | 100 | 100 | 100 | 100 | 100 | 100 |

**Key**: All numbers in the tables are wt% unless otherwise stated; QSP = sufficient quantity for 100%; [1] = Cellosize® HEC QP-4400H from Dow Europe GmbH; [2] = Keltrol® CG-T from CP Kelco A HUBER COMPANY; [3] = Genapol® C-100 from Clariant Produkte GmbH (Deutschland); [4] = Cremophor® EL from BASF The Chemical Company; [5] = 3-sulfopropyl acrylate potassium salt from Raschig.; [6] = Natrosol® 250 HHR from Herkules.

Ingredients 1 to 8 (up to and including citric acid) and mixed together first to create a first phase. The second phase consists of the following 6 ingredients (2-phenoxyethanol up to and including the fragrance). For the second phase, first the 2-phenoxyethanol and the p-hydroxybenzoic acid methylester are mixed until a solution is formed. Then 40°C coceth-10 is added to the solution and stirred. Subsequently PEG-35 castor oil and the fragrance are mixed in. The first and second phases are mixed together, and then the remaining ingredients are mixed together and the resulting composition mixed for 5 min.

COMPARATIVE DATA

Experiment 1 - Ethylenic monomer level

HPLC in combination with UV was used in order to measure the stability of the inhibited ethylenic monomer over time. The compositions were exposed to high-stress conditions i.e. high temperatures, as would be the case, for example, during shipment in a lorry without any air conditioning system. The incubation at high temperatures also can

aid prediction of the stability at RT for longer periods of time than measured here.

The HPLC column used was a Nucleodur C18 Pyramid, 5μm EC 250/4 (Macherey-Nagel) used at room temperature. The sample used was a 300 mg of a gel dissolved in 100 ml $H_2O$. The ethylenic monomer used was 3-sulfopropyl acrylate (3-SPA). 10 μl was the injection amount. Solvents A and B were used. Solvent A comprised: 990 ml $H_2O$, 10 ml $CH_3CN$, 2g $KH_2PO_4$, 1 ml $H_3PO_4$ (85%). Solvent B comprised: 400 ml $H_2O$, 600 ml $CH_3CN$, 2g $KH_2PO_4$, 1 ml $H_3PO_4$ (85%). The detector used was UV at 220 nm, which is able to detect inhomogeneities in the solvent flow, which corresponds to the ethylenic monomer.

The below table compares the levels of ethylenic monomer - in this case 3-SPA - after incubation at the indicated temperatures with the indicated inhibitors. The values refer to percentage of 3-SPA remaining. "Stopped" in the table indicates that due to poor performance, that composition was removed from the experiment and not analysed further.

| | Compound (b) | | | 4-methoxy phenol | | | Tocopherol | | |
|---|---|---|---|---|---|---|---|---|---|
| | RT | 60°C | 90°C | RT | 60°C | 90°C | RT | 60°C | 90°C |
| **Initial** | 100% | 100% | 100% | 100% | 100% | 100% | 100% | 100% | 100% |
| **2 wks** | 100%a | 100% | 100% | 100% | 100% | 100% | 100% | 75% | 10% |
| **4 wks** | 100% | 100% | 100% | 100% | 100% | 100% | 100% | 75% | 0% |
| **6 wks** | 100% | 88% | 25% | 100% | 77% | 15% | stopped | | |
| KEY: wks = weeks. | | | | | | | | | |

Conclusions from experiment 1: Ethylenic monomer inhibited by compound (b) i.e. pursuant to the present invention was stable for longer than ethylenic monomer inhibited by either 4-methoxy phenol or tocopherol. This was especially true for compound (b) versus the other inhibitors at 60°C and 90°C.

<u>Experiment 2 - Inhibitor Level at 60°C versus Time</u>

In this experiment, one inhibitor was added to a composition comprising an ethylenic monomer. The compositions were then incubated at 60°C for a number of weeks and the amount of inhibitor measured at the beginning and at the indicated time points. Inhibitor level measurement was also carried out by HPLC. The ethylenic monomer was 3-SPA. In some cases, different levels of the same inhibitor were compared. Compound (b) in this experiment was a mixture of 2-*tert*-butyl-4-hydroxy-anisole and 3-*tert*-butyl-4-hydroxy-anisole comprising circa 94%o to 99.99% 3-*tert*-butyl-4-hydroxy-anisole and circa 0.01% to 6% 2-*tert*-butyl-4-hydroxyanisole. Since 3-SPA is made from acrylic acid, the inhibitor for which is 4-methoxy phenol, then in all compositions tested, trace amounts of 4-methoxy phenol were present. "Stopped" in the table indicates that due to poor performance, that composition was removed from the experiment and not analysed further. In the below table the data is expressed as percentage inhibitor at 60°C versus time.

| Weeks | Initial | 1 | 2 | 4 | 6 | 8 |
|---|---|---|---|---|---|---|
| Inhibitor | % Inhibitor | % Inhibitor | % Inhibitor | % Inhibitor | % Inhibitor | % Inhibitor |
| 4-methoxy phenol (initial level: 226 mg/kg) | 100.0 | 95.6 | 91.2 | 85.4 | 79.2 | 72.6 |
| No inhibitor (trace quantities of 4-methoxy phenol remaining) (initial level: 31 mg/kg) | 100.0 | 100.0 | 100.0 | 80.6 | 77.4 | 71.0 |
| 4-methoxy phenol (initial level: 876 mg/kg) | 100.0 | 88.9 | 90.0 | 80.4 | 73.1 | 65.9 |
| alpha-Tocopherol (initial level: 666 mg/kg) | 100.0 | 69.1 | 62.5 | 39.6 | 33.0 | 26.3 |
| alpha-Tocopherol (initial level: 788 mg/kg) | 100.0 | 85.5 | 66.4 | 49.4 | 47.2 | 45.1 |
| tert-butylhydroquinone (initial level: 220 mg/kg) | 100.0 | 4.5 | stopped | stopped | stopped | stopped |

(continued)

| Weeks | Initial | 1 | 2 | 4 | 6 | 8 |
|---|---|---|---|---|---|---|
| Caffeic acid (initial level: 725 mg/kg) | 100.0 | 20.7 | 7.9 | stopped | stopped | stopped |
| Vitamin K3 (menadione) (initial level: 9 mg/kg) | 100.0 | n.b. | stopped | stopped | stopped | stopped |
| Ionol® 46 from Raschig GmbH (initial level: 624 mg/kg) | 100.0 | 69.2 | 51.3 | 40.1 | 30.6 | 21.8 |
| 2-Ethylhexyl-4-methoxycinnamate (initial level: 634 mg/kg) | 100.0 | 97.0 | 116.4 | 106.9 | 97.5 | stopped |
| delta-Tocopherol (initial level: 615 mg/kg) | 100.0 | 98.5 | 97.9 | 97.6 | 97.3 | - |
| Compound (b) (initial level: 704 mg/kg) | 100.0 | 91.6 | 83.4 | 84.8 | 86.2 | 88.1 |
| 4-Hydroxy-3-methoxycinnamic acid (initial level: 285 mg/kg) | 100.0 | 11.6 | stopped | stopped | stopped | stopped |
| alpha-Tocopherol (initial level: 146 mg/kg) | 100.0 | 108.2 | - | 75.3 | - | - |
| alpha-Tocopherol (initial level: 383 mg/kg) | 100.0 | 67.9 | 59.0 | 42.0 | - | - |

Experiment 3 - Inhibitor Level at 100°C versus Time

Exactly as per experiment 2, but incubation was carried out at 100°C. In the below table the data is expressed as percentage of inhibitor at 100°C versus time.

| Weeks Inhibitor | Initial % Inhibitor | 1% Inhibitor | 2% Inhibitor | 3% Inhibitor | 4% Inhibitor | 5% Inhibitor |
|---|---|---|---|---|---|---|
| 4-methoxy phenol (initial level: 226 mg/kg) | 100.0 | 40.7 | 30.5 | 20.8 | 15.5 | 11.1 |
| No inhibitor (trace quantities of 4-methoxy phenol remaining) (initial level: 31 mg/kg) | 100.0 | 58.1 | 54.8 | 51.6 | 29.0 | 16.1 |
| 4-methoxy phenol (initial level: 876 mg/kg) | 100.0 | 38.0 | 23.4 | 18.0 | 13.4 | 11.8 |
| alpha-Tocopherol (initial level: 666 mg/kg) | 100.0 | 6.2 | 1.5 | < 1.5 | < 1.5 | < 1.5 |
| alpha-Tocopherol (initial level: 788 mg/kg) | 100.0 | 13.1 | 7.1 | < 1.3 | <1.3 | <1.3 |
| tert-butylhydroquinone (initial level: 220 mg/kg) | 100.0 | <4.5 | stopped | stopped | stopped | stopped |
| Caffeic acid (initial level: 725 mg/kg) | 100.0 | 1.0 | 1.0 | stopped | stopped | stopped |
| Vitamin K3 (menadione) (initial level: 9 mg/kg) | 100.0 | - | stopped | stopped | stopped | stopped |

(continued)

| Weeks Inhibitor | Initial % Inhibitor | 1% Inhibitor | 2% Inhibitor | 3% Inhibitor | 4% Inhibitor | 5% Inhibitor |
|---|---|---|---|---|---|---|
| Iono® 46 from Raschig GmbH (initial level: 624 mg/kg) | 100.0 | 2.2 | <4.8 | 5.8 | <4.8 | 6.6 |
| 2-Ethylhexyl-4-methoxycinnamate (initial level: 634 mg/kg) | 100.0 | 71.0 | 48.1 | - | 18.9 | stopped |
| delta-Tocopherol (initial level: 615 mg/kg) | 100.0 | 55.8 | 35.6 | 27.5 | 19.2 | stopped |
| Compound (b) (initial level: 704 mg/kg) | 100.0 | 68.0 | 45.3 | - | 24.0 | 19.2 |
| 4-Hydroxy-3-methoxycinnamic acid (initial level: 285 mg/kg) | 100.0 | <3.5 | stopped | stopped | stopped | stopped |
| alpha-Tocopherol (initial level: 146 mg/kg) | 100.0 | 34.2 | <6.8 | <6.8 | <6.8 | stopped |
| alpha-Tocopherol (initial level: 383 mg/kg) | 100.0 | 23.2 | < 2.6 | <2.6 | <2.6 | stopped |

Conclusions of experiments 2 and 3 include: Compound (b) is more stable than the other inhibitors tested at elevated temperatures and for longer periods of time.

Experiment 4 - GPC

In an embodiment, during use, the ethylenic monomer polymerises. The molecular weights of polymer resultant from the polymerisation of ethylenic monomers may be obtained by routine GPC in water-based (anionic) solution. The GPC equipment comprises a single-channel degassing system [WGE-Dr.Bures], an isocratic pump [P 1000 of Spectra Physics], GPC-liquid 0.1 mol/l $NaNO_3$ at a flux rate of 1.0 ml/min, auto-sampler AS 1000 of Spectra Physics, sampling 100μl, PSS SUPREMA column (1) Guard (8 x 50 mm), (2) Suprema 3000 A (8 x 300 mm; 10μm), (3) Suprema 1000 A (8 x 300 mm; 10μm), (4) Suprema 100 A (8 x 300 mm; 10μm), column-oven K-7 of TECHLAB GmbH, Germany [at T=30°C], a UV -Detector UV 2000 of Spectra Physics, an RI-Detector Refractometer SEC-2010 of WGE Dr. Bures, Germany. Calibration was performed with Pullulan-Standards over a molecular weight range of 0.3 to 710 kDa. Data analysis was done using software WinGPC Unity by PSS.

The GPC data is presented in Figure 1. The x-axis represented by the letter 'x' is the molecular weight in Daltons (Da). The y-axis represented by the letter 'y' is the intensity. 3-SPA inhibited by 4-methoxy phenol i.e. not per the invention is indicated by the white diamonds. 3-SPA inhibited with compound (b) i.e. per the invention is indicated with black dots.

It is known in the art that the resulting polymer molecular weight is inversely proportional to the square root of the initial radical concentration - a radical in this sense being an ethylenic monomer having been activated by an initiator. Thus, it is important to understand the kinetics of ethylenic monomer consumption in order to make reliable conclusions from the GPC data. Hence, the applicant has utilised $^1$H-NMR to follow the kinetics of ethylenic monomer conversion. Conclusions drawn from Figure 1 thus include that a slight increase in molecular weight of polymer results when the composition of the present invention is utilised compared to a comparative composition.

Experiment 5 - $^1$H-NMR

The kinetics of the free radical polymerisation of ethylenic monomer in aqueous media can be followed using $^1$H-NMR. For example, the polymerisation of potassium 3-sulphopropylacrylate is shown in Reaction X below (wherein R-O-O-R is the initiator and n is the number of units polymerised).

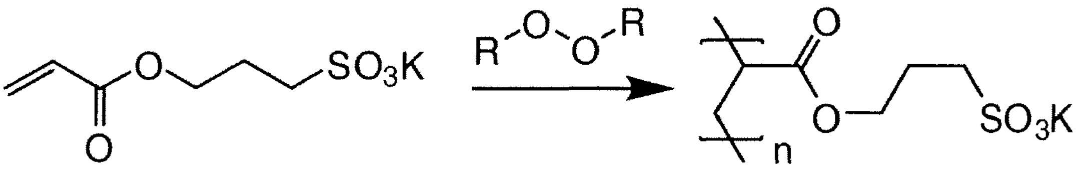


Reaction X

Determination of the conversion may be performed by comparison of the signals of the residual (unpolymerised) ethylenic monomer to a signal of a reference substance added to the reaction mixture before start of the reaction and/or by comparison to signals stemming from both polymer and ethylenic monomer. Triethylene glycol and sodium benzenesulfonate can be used as inert reference substances. The conversion is deduced by comparison of the integrals. When an inert reference substance is added, the conversion can be calculated according to Equation A:

$$\mathrm{conversion}[\%] = \left(1 - \frac{\dfrac{i_{mono}}{n_{H,mono} \cdot X_{mono}}}{\dfrac{i_{ref}}{n_{H,ref} \cdot X_{ref}}}\right) \cdot 100$$

Equation A

wherein $i_{mono}$: integral of the signal from ethylenic monomer; $n_{H,\ mono}$: number of H atoms in signal from ethylenic monomer; $X_{mono}$: amount of ethylenic monomer before start of the reaction in moles; $i_{ref}$: integral of the signal from reference; $n_{H,\ ref}$: number of H atoms in signal from reference; $X_{ref}$: amount of reference in moles.

For calculation of the conversion by comparison of residual ethylenic monomer signals and combined ethylenic monomer and polymer signals, Equation B can be utilised:

$$\mathrm{conversion}\,[\%] = \left(1 - \frac{\dfrac{i_{mono}}{n_{H,mono}}}{\dfrac{i_{comb}}{n_{H,comb}}}\right) \cdot 100$$

Equation B

wherein $i_{mono}$: integral of the signal from the ethylenic monomer; $n_{H,\ mono}$: number of H atoms in signal from ethylenic monomer; $i_{comb}$: integral of the combined signal from ethylenic monomer and polymer; $n_{H,\ comb}$: number of H atoms in combined signal from ethylenic monomer and polymer.

The following experimental procedure may be employed: ethylenic monomer and additives (when appropriate) in a round flask fitted with a silicone stopper are dissolved in 18 ml of the appropriate solvent (buffer with or without addition of salt, or ultra-pure demineralised water). The buffer that can be utilised for experiments at pH 5.8 is prepared as follows: 119.2 mg of $NaH_2PO_4$ and 141.9 mg of $Na_2HPO_4$ is dissolved in 110 ml of ultra pure water, then the pH is adjusted to the final value of 5.8 by addition of diluted phosphoric acid. The desired amount of initiator is dissolved in some ml of the same solvent (exact amount of solvent is adjusted individually to bring the total weight of the reaction mixture to 25.0 g). The reaction temperature is adjusted by an oil bath with contact thermometer. The reaction is started by transfer of the initiator solution to the reaction mixture with a syringe. In case no initiator is used, the flask with the starting materials is kept outside the oil bath before the reaction and the moment of contact with the oil bath is regarded as the starting point. At defined points in time, samples of 3.0 ml are drawn with a syringe. To stop the reaction, these samples are immediately exposed to air and 0.100 g of hydroquinone is added. The samples are freeze-dried separately and analysed by $^1$H-NMR spectroscopy for the determination of the conversion.

As an example, the structure of 3-sulfopropyl acrylate potassium salt is indicated below in Formula M, wherein

the $H_1$ indicates the position of the hydrogen atom that can be analysed by NMR.

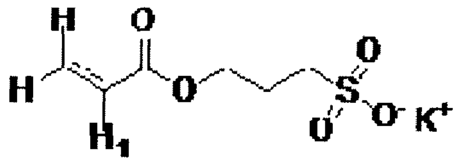

Formula M

Signals at 6.5 to 6.4 ppm (parts per million) corresponds to $H_1$ atom in the residual ethylenic monomer. Signals at 3.8 to 3.6 ppm corresponds to 12 H atoms in triethylene glycol. A signal at 4.4 to 4.2 ppm corresponds to the combined signal.

Utilising this technique, Figure 2 demonstrates the conversion of ethylenic monomer species into polymer species. The y axis labelled "y" shows conversion in percentage by mole of ethylenic monomer and the x-axis, which is labelled 'x', shows time in mins. For the experiment where the ethylenic monomer is inhibited with 4-methoxy phenol, the data points are labelled with black crosses joined by a dotted line. For the experiment where the ethylenic monomer is inhibited with compound (b) i.e. according to the present invention, the data points are labelled with open circles joined by a solid line. In both experiments, the compositions comprised: 4 wt% $Al_2(SO_4)_3$ x $18(H_2O)$ i.e. aluminium sulfate octadecahydrate; 12 wt% 3-sulfopropyl acrylate as ethylenic monomer, 127 mg cysteine hydrochloride. Further reaction conditions included: carried out in ambient atmosphere, at pH 5.8, utilising 25.8 mmoles of hydrogen peroxide as initiator.

Conclusions drawn from Figure 2 include that polymerisation occurs such that almost all ethylenic monomer is converted and that there is no clear difference in reaction kinetics when compositions pursuant and not pursuant to the present invention are compared. Compound (b) results in parity reaction kinetics versus 4-methoxy phenol.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A personal care composition comprising:

   (a) an ethylenic monomer having a molecular weight of 500 g/mole or less;
   (b) a compound selected from the group consisting of: 2-*tert*-butyl-4-hydroxy-anisole, 3-*tert*-butyl-4-hydroxy-anisole, and mixtures thereof;
   wherein (b) is present in the composition in an amount of from about 1 milligram to about 1000 milligram per kilogram of (a).

2. The composition according to claim 1, wherein the composition comprises less than about 500 ppm, or less than about 400 ppm, or less than about 300 ppm, or less than about 200 ppm, or less than about 100 ppm, or less than about 50 ppm, or less than about 30 ppm, of 4-methoxy phenol.

3. The composition according to any of the preceding claims, wherein the composition further comprises a cation and an anion; wherein the cation is selected from the group consisting of inorganic cations having a charge density of 0.05 charge/picometre or more.

4. The composition according to any of the preceding claims, wherein the composition further comprises a cosmetically acceptable carrier.

5. The composition according to any of the preceding claims, wherein the composition further comprises a viscosity-increasing agent, wherein the viscosity-increasing agent comprises at least one polysaccharide, preferably at least one heteropolysaccharide.

6. The composition according to any of the preceding claims, wherein (b) is present in the composition in an amount of from about 50 milligram to about 300 milligram, or from about 100 milligram to about 200 milligram, per kilogram

of (a).

**7.** The composition according to any of the preceding claims, wherein the ethylenic monomer is present in an amount of from about 0.1% to about 20%, or from about 1% to about 15%, or from about 5% to about 14%, or from about 7% to about 13%, by total weight of the composition.

**8.** The composition according to any of the preceding claims, wherein the ethylenic monomer is selected from the group consisting of: mesaconic acid, 2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2-methylenebutyrate, methacryloyl-L-lysine, N-(2-hydroxypropyl)methacrylamide, 2-acrylamidodiglycolic acid, 2-ethoxyethyl acrylate, 2-butoxyethyl acrylate, N-isopropylmethacryalmide, 2-aminoethyl methacrylate, 2-bromoethyl acrylate, 3-(dimethylamino)propyl acrylate, (3-acrylamidopropyl)trimethyl ammonium salt, [2-(acryloyloxy) ethyl]-trimethylammonium salt, alkylacetamidoacrylate, sulfoalkyl (meth)acrylate, 3-sulfopropyl acrylate, 3-sulfopropyl methacrylate, derivatives thereof, and mixtures thereof.

**9.** The composition according to any of the preceding claims, wherein the composition is substantially free of a polymer derived from the polymerisation of ethylenic monomers.

**10.** The composition according to any of the preceding claims, wherein the composition is substantially free of a polymerisation inhibitor, with the exception of compound (b).

**11.** A method of treating hair and/or skin comprising applying the composition, according to any preceding claim, onto hair and/or skin.

**12.** The method according to claim 11, wherein the hair and/or skin has been pre-treated with an oxidising formulation.

**13.** A kit comprising:

(i) a composition, according to any of claims 1 to 10;
(ii) an oxidising formulation.

**14.** Use of the composition, according to any of claims 1 to 10, for treating hair and/or skin.

**15.** Use, according to claim 14, for modifying the internal region of a hair shaft.

Fig. 1

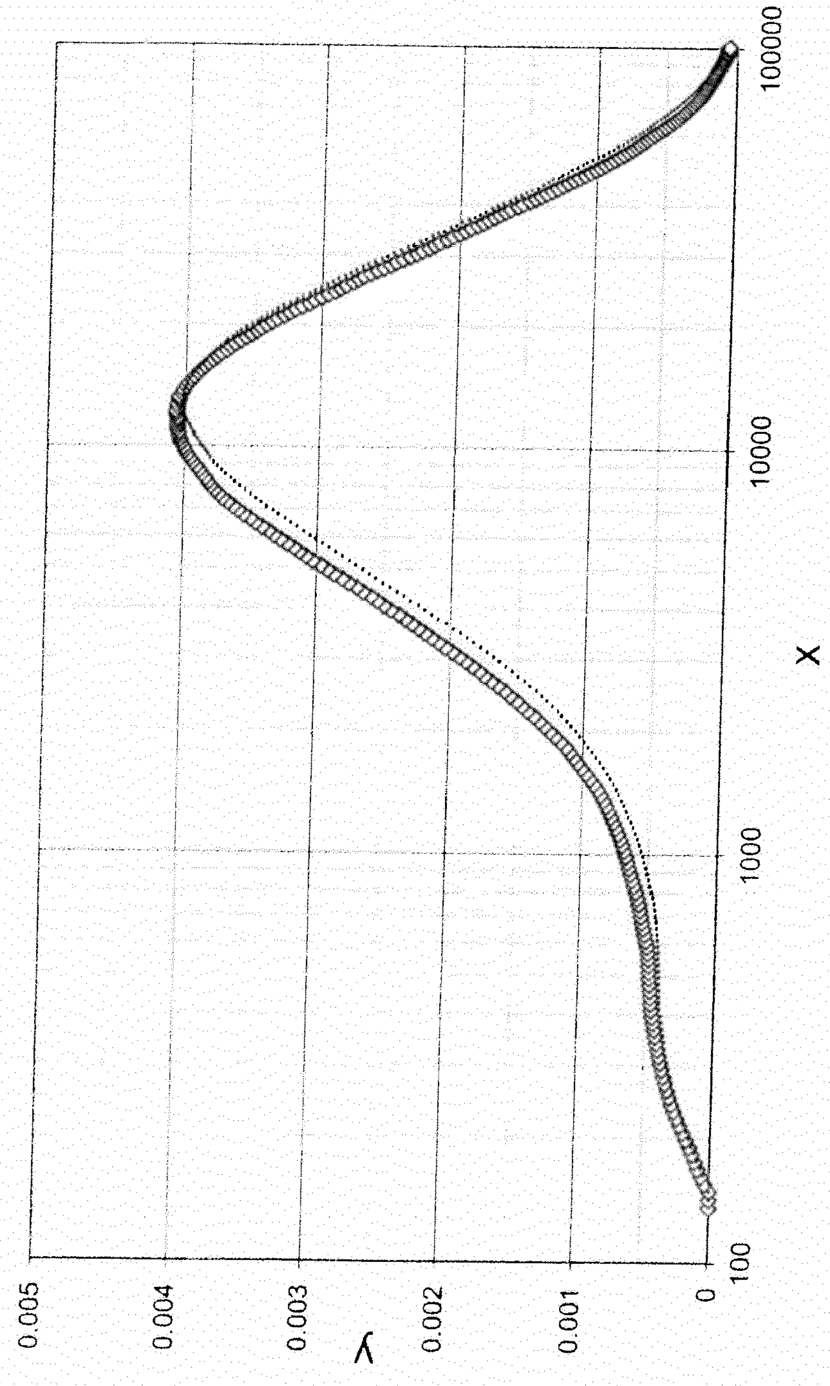

Fig. 2

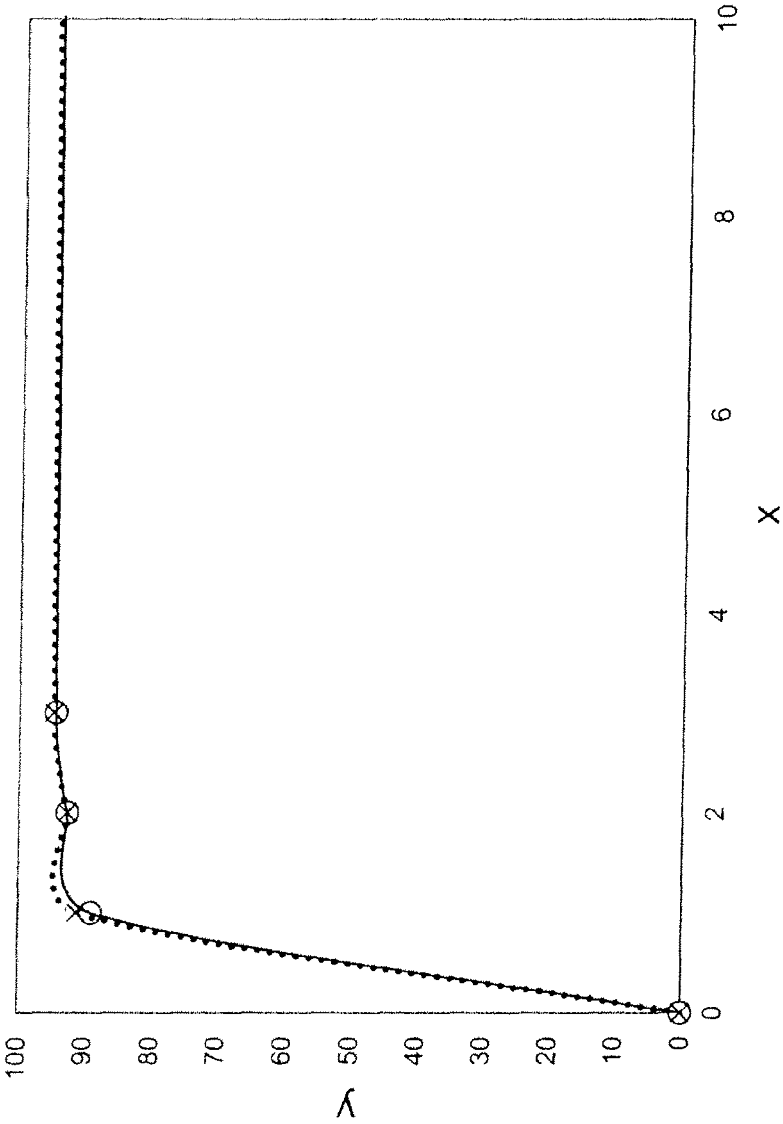

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009088520 A1
- EP 11151384 A


### Non-patent literature cited in the description


- **ATKINS P.W.** Physical Chemistry. 2001
- International Cosmetic Ingredient Dictionary. 2002
- CTFA Cosmetic Ingredient Handbook. 2004